# EUROPEAN PATENT SPECIFICATION

(11) **EP 1 144 010 B1**
(45) Date of publication and mention of the grant of the patent: **18.04.2007**
(21) Application number: 00904230.0
(22) Date of filing: 06.01.2000
(51) Int. Cl.: A61K 47/48, A61K 39/395, A61K 38/17, A61K 38/13, A61K 31/4353, A61K 31/52, A61K 31/56, A61P 37/06

(54) **PREVENTION OF CHRONIC GRAFT REJECTION BY A COMBINATION OF IMMUNOTOXINS AND COSTIMULATION BLOCKERS**
VORBEUGUNG DER KRONISCHEN TRANSPLANTAT-ABSTOSSUNG DURCH EINE KOMBINATION VON IMMUNTOXINEN UND KOSTIMULATIONBLOCKER
PRÉVENTION DU REJET CHRONIQUE DE GREFFE PAR UNE COMBINAISON D'IMMUNOTOXINES ET DE BLOQUEURS DE LA COSTIMULATION

(30) Priority: 08.01.1999 US 115252 P
(43) Date of publication of application: 17.10.2001
(73) Proprietor: WISCONSIN ALUMNI RESEARCH FOUNDATION, Madison, WI 53705 (US)
(72) Inventor: KNECHTLE, Stuart, J., Oregon, WI 53575 (US); KIRK, Allan, D., Potomac, MD 20854 (US)
(74) Representative: Leissler-Gerstl, Gabriele
(86) International application number: PCT/US2000/000284
(87) International publication number: WO 2000/040270

(56) References cited:
- WO-A-95/34320
- WO-A-96/32137
- WO-A-98/39363
- WO-A-98/52606
- WO-A-98/56417
- WO-A-99/53954
- KOULMANDA M ET AL: "Cyclophosphamide, but not CTLA4Ig, prolongs survival of fetal pig islet grafts in anti-T cell monoclonal antibody-treated NOD mice." XENOTRANSPLANTATION (1998) 5 215-21 , XP000915296

## Description

### BACKGROUND OF THE INVENTION

### Field of the Invention

The present invention relates to use of an Immunotoxins in conjunction with costimulation blockade as a means for preventing immune-mediated chronic rejection of a transplant. More specifically, the present invention relates to the blocking of CD40 ligand (CD154) and CD28 in conjunction with immunotoxin to prevent rejection.

### Background Art

Activated T cells play a central role in the immune response to antigens by regulating the function of immune cells including B cells, the sole source of Ab in vivo. The main route for T cell activation in vivo is the interaction between the T cell receptor (TCR) complex and its respective Ag-MHC, presented by antigen presenting cells (APC). Ag-bound TCR initiates a cascade of intracellular reactions that leads to the release of cytokines and to the increase in the expression of surface molecules. T cells' cytokines activate a variety of cells, including macrophages and B cells, by binding to specific receptors on those cells. In addition to TCR-Ag binding, other molecules on T cells (i.e., CD154 and CD28) bind to their counterparts on APC (i.e., CD40 and B7, respectively) leading to the generation of regulatory intracellular signals not only in T cells but also in APC. Indeed, such interactions have been shown to profoundly influence the function of immune cells, and, in turn, the immune response to antigens (Schwartz, R.H. (1996); Boussiotis et al. (1994); Lenschow et al. (1992)).

Chronic graft rejection is characterized by an insidiously progressive loss of function of the grafted organs. This form of rejection is slow and begins many months or even years after transplantation. Although the role of Ab in chronic rejection is unclear since Ab to donor antigens are found in some but not all patients, donor directed antibodies have been strongly implicated as a contributing cause of chronic rejection (Sayegh and Turka (1998)). The presence of anti-graft Ab is likely to accelerate the rejection process by binding to the endothelium, and in turn, causing cell damage by fixing complement and/or mediating cell-dependent cytotoxicity (ADCC). Mediators released from injured endothelial cells could attract a variety of cells to the injured vascular endothelium leading to more tissue destruction. The increase in the expression of several adhesion molecules on the injured endothelium and on accumulating cells assists in localizing infiltrating cells at the site of injury. Consequently, the damaged endothelium is covered by a layer of platelets and fibrin, and eventually by proliferating cells. The end result is a proliferative lesion in the vessels progressing toward fibrosis and occlusion.

Anti-T cell immunosuppressive strategies do not prevent antibody-mediated graft injury or chronic rejection. Although it has been shown previously in the rhesus monkey renal allograft model that a toxin conjugated anti-CD3 antibody (IT) substantially promotes allograft survival, most recipients develop chronic rejection with characteristics of immune mediated injury. This may be due to the fact that significant T cell depletion by the IT requires 48 hours, a period that may permit T cells to activate B cells. Thus, antibody-mediated chronic rejection remains one of the greatest problems in clinical organ transplantation, and methods of preventing this antibody-mediated chronic rejection are still needed.

### SUMMARY OF THE INVENTION

The present invention provides the use of a combination as defined in claim 1 for preventing chronic rejection of a transplant in a recipient. This is achieved by administering to the recipient an immunotoxin, thereby reducing the recipient's T-cell population and administering to the recipient a costimulation blocker, thereby reducing a transplant-specific antibody response. The immunotoxin used in the present invention can be a divalent anti-T cell immunotoxin directed at the CD3 epitope. The combination for use in the present invention can further comprise a second costimulation blocker, for further reducing the transplant-specific antibody response. Thus, the costimulation blockers used in the present invention can include a CD40:CD154 pathway blocker (for example, 5C8) and/or a B7:CD28 pathway blocker (for example, CTLA 4-Ig). Also provided is the preventing chronic rejection of a transplant in a recipient, further comprising an immunosuppressive agent being administered to the recipient. A method of preventing chronic rejection comprises monitoring the recipient for an indicator of a late acute rejection and administering to the recipient showing the indicator of the late acute rejection an immunotoxin, thereby reducing the recipient's T-cell population.

### DETAILED DESCRIPTION OF THE INVENTION

The present invention provides the use of a combination as defined in claim 1 for preventing chronic rejection of a transplant in a recipient. This is achieved by administering to the recipient an immunotoxin, thereby reducing the recipient's T-cell population and administering to the recipient a costimulation blocker, thereby reducing a transplant-specific antibody response. Immunotoxin administration to prevent acute transplant rejection requires up to 48 hours to kill T lymphocytes; however, T helper cell participation and B cell activation occur within the first 48 hours and commit the host to an anti-donor antibody response. During this activation period, costimulation blockers prevent B cell sensitization and avert late antibody-mediated rejection. The present use prevents chronic rejection of a transplant by a recipient by inducing immune tolerance in the recipient. Immune tolerance is achieved without the need for administration of donor cells, such as donor bone marrow cells or splenocytes, to the recipient.

"Costimulation" refers to the non-antigen specific activation of T cells, which occurs in parallel with antigen specific stimulation. Many T-cell molecules serve as receptors for costimulatory signals, including, for example, CD28, CTLAa-4, and CD40 ligand. CD28 has two known ligands, B7-1 (CD80) and B7-2 (CD86), which are expressed on activated antigen-presenting cells. CTLAa-4 also binds B7-1 and B7-2 but, unlike CD28, terminates the immune response. The CD40:CD154 pathway also plays a role in T-cell costimulation. An example of CD40 ligand is CD154, which is expressed on activated CD4 T cells. Stimulation of CD40 promotes antibody production by B cells, induces expression of adhesion molecules and cytokines that activate T cells, and induces B7 expression by antigen presenting cells. In the absence of costimulation, a T cell that encounters an antigen does not produce appreciable amounts of cytokines and does not divide. The T cell lacking costimulation, thus, becomes unresponsive to stimulation and undergoes programmed cell death.

Because activated T cells regulate the function of other immune cells, T cell unresponsiveness will influence the immune response to various antigens. CD154-CD40 interaction regulates antibody production from B cells, the only source of antibody in vivo. Therefore, costimulation blockade, when given to a transplant recipient in combination with an anti-T cell IT influences the responsiveness of immune cells to stimuli.

The costimulation blocker used in the present invention can block the CD40:CD154 pathway. More specifically, the blocker of the CD40:CD154 pathway can be an anti-CD154, including, for example, 5C8, which has been described in detail previously (Lederman, Yellin, Krichevsky et al. (1992); Lederman, Yellin, Inghirami et al. (1992)). 5C8 has subsequently been humanized and is available commercially (Biogen). Other CD40:CD154 antibodies can be used.

Alternatively, the costimulation blocker of the present invention can block the B7:CD28 pathway. More specifically, the blocker of the B7:CD28 pathway can bind to B7 and block the binding of B7 with CD28. More specifically, the blocker of the B7:CD28 pathway is CTLA4-Ig, which has been described previously (Lin et al. (1993); Sayegh MH et al. (1997); Pearson et al. (1997)). CTLA4-Ig is commercially available (e.g., Bristol-Meyers, Squib, Repligen Corp.). Other blockers of the B7:CD28 pathway include, for example, anti-CD80 and anti-CD86, which are available from Genetics Institute.

As used herein, "rejection" refers to immunologic rejection of a transplanted organ by a host. "Chronic rejection" of a transplant, generally occurs months or years after transplantation, most often occurring at least six months after transplantation, and is characterized histologically (for example, upon biopsy of the transplant) by such characteristics as interstitial fibrosis, interstitial hyperplasia, arteriolar narrowing, and ischemic injury to the transplanted organ. Additionally, histological signs of chronic rejection of a kidney transplant include reduplication and thickening of the glomerular basement membrane. The clinical signs of chronic rejection include gradually progressive graft dysfunction. For example, in a subject with a kidney transplant, gradually increasing blood levels of creatinine would indicate chronic rejection. Normal serum levels of creatinine are less than 2.0 mg/dl, and more typically about 0.5 to 1.5 mg/dl. Renal graft dysfunction is evidenced by approximately a two fold increase in baseline levels of creatinine. The presence of antidonor antibody and increasing levels of antidonor antibody could also indicate graft dysfunction. One skilled in the art would recognize other histological indicators and clinical indicators of chronic rejection. Chronic rejection may be mediated by both alloantigen-dependent and alloantigen-independent mechanisms.

By "reducing a transplant-specific antibody response" is meant decreasing or eliminating the antidonor antibodies in the transplant recipient. A reduction in a transplant-specific antibody response can be assessed by measuring the circulating levels of antidonor antibodies. Preferably, the reduction in the transplant-specific antibody response would be characterized by an alloantibody level in the transplant host of less than about 50% of control levels. More preferably, the alloantibody levels will be less than about 40%, 30%, 20%, 10%, or 5% of control. Even more preferably, the reduction in the transplant-specific antibody response would be an elimination of the alloantibody response so that no antidonor antibodies are present in the host.

By "reducing the recipient's T-cell population" is meant a transient reduction in the recipient's T cells in blood and lymph nodes. Preferably, the immunotoxin transiently reduces the recipient's T cells in the blood and lymph nodes by at least one log unit.

Further provided is the use of the present invention, wherein the transplant is allogeneic. The recipient can be a mammal, and more specifically a primate. Preferably, the primate recipient is a human.

In the present use, the transplant can be derived from either a living donor or a cadaveric donor. Preferably, the transplant is selected from the group consisting of kidney, lung, heart, liver, pancreas, or skin. The transplant can be a whole organ or a part thereof. Thus, for example, the pancreatic transplant can be pancreatic islet cells.

The combination for use in the present invention can further comprise a second costimulation blocker, for further reducing the transplant-specific antibody response. If the first costimulation blocker blocks the CD40:CD154 pathway, the second costimulation blocker preferably blocks the B7:CD28 pathway. The blocker of the B7:CD28 pathway can be, for example, CTLA 4-Ig. CTLA4-Ig binds to B7-1 and B7-2, thereby blocking the B7 interaction with CD28 on T cells. Thus, two costimulation blockers can be used to further reduce the transplant-specific antibody response.

Alternative embodiments of the present invention include the use in a variety of treatment regimens. In one group of embodiments is a short treatment regime, wherein immunotoxin and costimulation blockers are administered during the period following transplantation and then discontinued. In another group of embodiments, administration of costimulation blockers, rather than being discontinued following the short treatment regimen, is continued at regular intervals. In a final group of embodiments, a series of immunotoxin administrations are repeated subsequent to the short treatment regimen or during the ongoing treatment with costimulation blockers.

According to the use of the present invention, the immunotoxin is administered at least three times. Preferably, the immunotoxin is administered at least on the day of transplanting and on two days immediately following transplanting. The costimulation blocker or combination of blockers is administered at least once, preferably on the day of transplantation. The costimulation blocker or combination of blockers can be administered at least on three additional days within the first week following transplantation or every other day for two weeks. Alternatively, treatment with the costimulation blocker can be ongoing, e.g., administrations every other day, weekly, or monthly for the life of the transplant recipient.

It is understood that late B cell activation may occur if T cells recover and repopulate (following death by IT) or if T cells escape costimulation blockade over time, thus necessitating ongoing administration with either 5C8, CTLA4-Ig, or a combination thereof. Thus, costimulation blockers could be administered monthly after the original treatment or series of treatments as a preventative. Alternatively, "late" doses of costimulation blockade can be administered to rescue transplants from chronic rejection if anti-donor antibody titers rise or other indicators of chronic rejection occur at late time points. Thus, the present invention provides a method further comprising costimulation blockade for at least one week, two weeks, three weeks, four weeks, five weeks, six weeks, seven weeks, eight weeks, nine weeks, ten weeks, eleven weeks, twelve weeks, or longer. The costimulation blocker can be administered every other day, weekly, or monthly. The additional costimulation blockade can be administered immediately after a first treatment or series of treatments or can be delayed until anti-donor antibody titers increase or other indicators of chronic rejection occur. One skilled in the art would know that the costimulation blocker could be administered at regular intervals after the original treatment regimen. For instance, since 5C8 has an approximately 35 day half-life, it could be redosed monthly to maintain costimulation blockade. Regimens using other costimulation blockers can be determined using similar criteria.

Treatment regimens can include administration of the costimulation blocker, for example, CTLA4-Ig or 5C8, in combination with a 3-day course of IT. The costimulation blocker or combination of costimulation blockers can be administered as a brief one week course or longer. Examples of treatment regimens include the following:
Regimen 1: IT day 0, 1, 2 (0.2 mg/kg) + 5C8 day 0, 2, 4, 6, 8, 12, 16, 28 (20 mg/kg/day);
Regimen 2: IT day 0, 1, 2 (0.2 mg/kg) + 5C8 day 0, 2, 4, 6 (20 mg/kg/day);
Regimen 3: IT day 0, 1, 2 (0.2 mg/kg) + CTLA4-Ig day 0, 2, 4, 6, 8, 12, 16, 28 (20 mg/kg/dose);
Regimen 4: IT day 0, 1, 2 (0.2 mg/kg) + CTLA4-Ig day 0, 2, 4, 6 (20 mg/kg/dose);
Regimen 5: IT day 0, 1, 2 (0.2 mg/kg) + 5C8 day 0, 2, 4, 6, 8, 12, 16, 28 (20 mg/kg) + CTLA4-Ig day 0, 2, 4, 6, 8, 12, 16, 28 (20 mg/kg/dose); and
Regimen 6: IT day 0, 1, 2 (0.2 mg/kg) + 5C8 day 0, 2, 4, 6 (20 mg/kg/dose) + CTLA4-Ig day 0, 2, 4, 6 (20 mg/kg/dose).

One skilled in the art would know that the doses of IT, 5C8, CTLA4-Ig could be decreased in a stepwise manner. For example, if signs of serious infection risk (such as cytomegalovirus infection, fever, hypotension, or bacterial or fungal infection), lymphoproliferative disease, or other malignancy occur, the doses could be decreased. Similarly, if CD4 counts, as measured by flow cytometry or other means, decrease, then doses could be decreased. Thus, the dose of IT could be decreased to 0.19, 0.18, 0.17, 0.16, 0.15, 0.14, 0.13, 0.12, 0.11, 0.10, or 0.05 mg/kg. The dose of 5C8 or CTLA-4-Ig could be decreased to 15, 10, 5, 2, or 1 mg/kg/dose.

Also provided is the use of a combination as defined above for preventing chronic rejection of a transplant in a recipient, further comprising an immunosuppressive agent. Preferably, the immunosuppressive agent is selected from the group consisting of cyclosporine (e.g., cyclosporine A), cyclophylins, mycophenolate moefitil, tacrolimus, azathioprine, and steroid (e.g., methyl prednisone), and any combination thereof, or other immunosuppressant known in the art. Thus, a method can include administering an immunosuppressant compound before, at the same time, or after the immunotoxin and costimulation blocker steps. Certain of these immunosuppressants have major effects on cytokine release occurring in the peritransplant period that may aid in the induction of the tolerant state.

Another embodiment of the invention involves subsequent use of an immunotoxin to rescue a transplant from a late acute rejection. Thus, the present invention provides a method of reversing a late acute rejection of an transplant by a recipient having a transplant that has survived for a prolonged period of time. The method calls for using the method of preventing chronic rejection as described herein and further comprises monitoring the recipient for an indicator of a late acute rejection and administering to the recipient showing the indicator of the late acute rejection an immunotoxin, thereby reducing the recipient's T-cell population. Thus, when signs of an acute rejection occur, a second short course of immunotoxins can be administered. For example, three doses of immunotoxin for a total dose of 0.2 mg/kg can be administered when signs of acute rejection occur. The subsequent treatment with immunotoxin optionally can be accompanied by a second course of costimulation blockers or by the ongoing treatment with costimulation blockers.

"Acute rejection," as used herein, refers to a phenomenon that is predominantly T lymphocyte mediated and that generally occurs within hours, days, weeks, or months of transplantation, most often occurring within about the first six months after transplantation. Acute rejection is characterized histologically by a prominent lymphocyte infiltration. The lymphocyte infiltration is seen, for example, in the renal tubules (i.e., tubulitis) of a transplanted kidney and in the bile ducts of a transplanted liver. Arteritis is another histological sign of acute rejection. Clinical signs of an acute rejection would include signs of a rapid decrease in transplant function. For example, a sharp increase in serum creatinine would be associated with an acute rejection of a kidney transplant. Sharp increases in titers of antidonor antibody would also be associated with an acute rejection. One skilled in the art would recognize other clinical indicators of acute rejection.

"Late acute rejection" as used herein includes an acute rejection anytime during or after the course of treatment with immunotoxin or costimulation blockers. Thus, "late acute rejection" can include an acute response within about 3 days, 5 days, 1 week, 2 weeks, 3 weeks, 4 weeks, 6 weeks, 8 weeks, 10 weeks, 12 weeks, 14 weeks, 16 weeks, 5 months, 6 months, or longer after transplantation.

The immunotoxin can be a divalent anti-T cell immunotoxin. The immunotoxins have been described previously in U.S. Patent Nos. 5,167,956 and 5,725,857 and in PCT WO 98/39425 and PCT WO 98/39363. The immunotoxin can be directed at the CD3 epitope. More specifically, the divalent anti-T cell immunotoxin can comprise a toxin moiety and a targeting moiety directed to the T cell CD3ε epitope. The toxin moiety can be a diphtheria toxin. Thus, the divalent anti-T cell immunotoxin can be UCHT1-CRM9. More specifically, the divalent anti-T cell immunotoxin can be a single chain engineered fusion protein comprising an amino-terminus DT based toxin domain fused to a sFv domain (VL-linker-VH where linker is (Gly4Ser)3 separated by a second linker and fused to a second identical sFv domain. The second linker can be (Gly4Ser)3 or (Gly4Ser)5. Alternatively, the single chain engineered fusion protein can be monovalent providing that the linker and the VL and VH sequence are carefully chosen to provide an affinity lying within ± 0.5 log unit of the parental antibody affinity.

The divalent disulfide linked immunotoxin can have identical components. Alternatively, the components can be non-identical with only one toxin moiety to minimize stearic hindrance of the antigen binding domains. The divalent anti-T cell immunotoxin can be a disulfide dimer of two monovalent single chain engineered fusion proteins that are dimerized via the hinge region of IgG1 or the µCH2 domain of IgM. The dimer can be a homo dimer comprising two monovalent units of DT390-sFv-H-γCH3, disulfide dimerized by the single or double cysteine residues in H, the hinge region. The dimer alternatively can be a heterodimer comprising one monovalent unit of DT390-sFv-H-γCH3, disulfide dimerized by the single or double cysteine residues in H to a monovalent unit of sFv-H-γCH3. Dimerization can be achieved in vivo by expression in a eukaryotic expression system modified for toxin resistance by an EF2 mutation. Examples are mutated CHO cells, Ss9 insect cells, or mutated yeasts. Alternatively, dimerization can be performed in vitro from monovalent species produced in prokaryote expression systems by the use of disulfide interchange reactions employing suitable disulfide oxidation systems such as dithiobisnitrobenzoic acid used to generate mixed disulfide intermediates.

The toxin moiety can be a diphtheria toxin binding site mutant. The immunotoxin can comprise a mutant toxin moiety (e.g., DT toxin or ETA toxin) linked to a single chain (sc) variable region antibody moiety (targeting moiety). Thus, the invention utilizes an immunotoxin having recombinantly produced antibody moiety linked (coupled) to a recombinantly produced toxin moiety and a fusion immunotoxin (where both toxin and antibody domains are produced from a recombinant construct). As the application provides the necessary information regarding the arrangement of toxin and antibody domains, and the sub regions within them, it will be recognized that any number or chemical coupling or recombinant DNA methods can be used to generate an immunotoxin. Thus, reference to a fusion toxin or a coupled toxin is not necessarily limiting.

The antibody moiety preferably routes by the anti-CD3 pathway. The immunotoxin can be monovalent, but divalent antibody moieties are presently preferred since they have been found to enhance cell killing by about 3 to 15 fold. The immunotoxin can be a fusion protein produced recombinantly. The immunotoxin can be made by chemical thioether linkage at unique sites of a recombinantly produced divalent antibody (targeting moiety) and a recombinantly produced mutant toxin moiety. The targeting moiety of the immunotoxin can comprise the human µCH2, γCH3 and µCH4 regions and VL and VH regions from murine Ig antibodies. These regions can be from the antibody UCHT1 so that the antibody moiety is scUCHT1, which is a single chain CD3ε antibody having human *µ*CH2, γCH3 and µCH4 regions and mouse variable regions. These are believed to be the first instances of sc anti-CD3 antibodies. Numerous DT mutant toxin moieties are described herein, for example DT390 or extensions of DT390 out to DT530. Thus, as just one specific example the immunotoxin, the invention provides scUCHT1-DT390. Derivatives of this immunotoxin are designed and constructed as described herein.

The engineered divalent immunotoxin utilized in the present invention can be stabilized with respect to divalency and disulfide bond initiation by the interactive Ig domains of either human IgM µCH2 or IgG1 γCH3 or other similar Ig interactive domains.

In the present invention, the toxin moiety retains its toxic function and membrane translocation function to the cytosol in full amounts. The loss in binding function located in the receptor binding domain of the toxin protein diminishes systemic toxicity by reducing binding to non-target cells. Thus, the immunotoxin can be safely administered. The routing function normally supplied by the toxin binding function is supplied by the targeting antibody, for example, anti-CD3. The essential routing pathway is (1) localization to coated pits for endocytosis, (2) escape from lysosomal routing, and (3) return to the plasma membrane.

The mutant DT toxin moiety can be a truncated mutant, such as DT390, extensions of DT390 out to DT530, or other truncated mutants, as well as a full length toxin with point mutations or CRM9 (cloned in *C. ulcerans),* scUCHT1 fusion proteins with DTM1 and DT483. DT390 has been cloned and expressed in *E*. *coli.* The toxin domain can be CRM9 (DT535, S525F) plus a second attenuating mutation from the group: F530A, K516E, K516A, Y514A, V523A, N524A). The antibody moiety can be scUCHT1 or other anti-CD3 antibody having the routing and other characteristics described in detail herein. Thus, one example of an immunotoxin for use in the present methods is the fusion protein immunotoxin DT390sSvUCHT1. The described immunotoxins can be used in the invention.

The immunotoxin or components thereof can be expressed in *E*. *coli* BL21DE3 cytosol using TrxB-strains at 15 to 25°C. Alternatively, the immunotoxin or components thereof can be expressed in eukaryotic cell lines (such as CHO), insect cell lines (such as Ss9), and yeast cell lines (such as *Pichia pastoris)* provided that the toxin glycosylation sites at residues 16 and 235 are eliminated and the cells have been mutated to resist ADP-ribosylation catalyzed by toxin, by a Gly to Arg substitution 2 residues to the carboxyl side of the modified amino acid diphthamide. In the case of insect cells this mutant EF-2 can be supplied in the same baculovirus vector supplying the immunotoxin gene since two late promoters are available in baculovirus.

The recombinant immunotoxins can be produced from recombinant sc divalent antibody or recombinant dicystronic divalent antibody and recombinant mutant toxins each containing a single unpaired cysteine residue. An advantage of this method is that the toxins are easily produced and properly folded by their native bacteria while the antibodies are better produced and folded in eukaryote cells. In addition, this addresses differences in coding preferences between eukaryotes and prokaryotes which can be troublesome with some immunotoxin fusion proteins.

The general principles for producing the present divalent recombinant anti-T cell immunotoxins are:
1. The disulfide bond bridging the two monovalent chains is chosen from a natural Ig domain, for example from µCH2 (C337 of residues 228-340 or the γIgG hinge region, C226 or C229 or both of residues 216-238 [with C220P]).
2. Sufficient non-covalent interaction between the monovalent chains is supplied by including domains having high affinity interactions and close crystallographic or solution contacts, such as µCH2, µCH4 (residues 447-576) or γCH3 (residues 376-446). These non-covalent interactions facilitate proper folding for formation of the interchain disulfide bond.
3. For fusion immunotoxins the orientation of the antibody to the toxin is chosen so that the catalytic domain of the toxin moiety becomes a free entity when it undergoes proteolysis at its natural processing site under reducing conditions. Thus, in the ETA based IT, the toxin moiety is at the carboxy terminus and, in DT based fusion IT, the DT based toxin moiety is at the amino terminus of the fusion protein.
4. For chemically coupled immunotoxins, a single cysteine is inserted within the toxin binding domain. The antibody is engineered to have only a single free cysteine per chain which projects into the solvent away from interchain contacts such as µCH3 414, µCH4 575 or the addition to γCH3 at C447. Crystal structure indicates this region is highly solvent accessible. Excess free cysteines are converted to alanine. Alternatively, a C terminal cysteine can be added to γCH3 directly following a histidine tag for purification, γCH3 (His)6Cys.
5. Toxins are mutated in their binding domain by point mutations, insertions or deletions, have at least a 1000 fold reduction in binding activity over wild type, and are free of translocation defects.
6. Toxin binding site mutants, if not capable of proteolytic processing at neutral pH, are modified in the processing region to achieve this result.

A binding site mutant (CRM9) of full length diphtheria toxin residues 1-535 using the numbering system described by Kaczorek et al. (1983) S525F; (Shen et al. (1994)) can be further modified for chemical coupling by changing a residue in the binding domain (residues 379-535) to cysteine. Presently preferred residues are those with exposed solvent areas greater than 38%. These residues are K516, V518, D519, H520, T521, V523, K526, F530, E532, K534 and S535 (Shen et al. (1994)). Of these K516 and F530 are presently preferred since they are likely to block any residual binding activity (Shen et al. (1994)). However, maximal coupling of the new cysteine residue will be enhanced by the highest exposed solvent surface and proximity to a positively charged residue (which has the effect of lowering cysteine -SH pKa). These residues are at D519 and S535 so that these are presently preferred from the above list of possibilities.

A double mutant of DT containing the S525F mutation of CRM9 plus an additional replacement within the 514-525 exposed binding site loop to introduce a cysteine coupling site for example T521C can be produced in Corynebacterium ulcerans preceded by the CRM9 promoter and signal sequence. The double mutant is made in Corynebacterium ulcerans by a recombination event between the plasmid producing CRM9-antibody fusion protein and PCR generated mutant DNA with a stop codon at 526 (gapped plasmid mutagenesis). Alternatively, double attenuating mutants of diphtheria toxin can be produced in *E. coli* BL21DE3 TrxB by PCR mutagenesis without the use of a signal sequence. These CRM9-Cs can be used to form specific thioether mutant toxin divalent antibody constructs by adding excess bismaleimidohexane to CRM9-Cs and coupling to single chain divalent antibody containing a free cysteine at either the end of the µCH4 domain or the γCH3 domain (see Serial No. 081739.703).

These and other mutations are accomplished by gapped plasmid PCR mutagenesis (Muhlrad et al. (1992)) using the newly designed *E*. *coli*/*C. ulcerans* shuttle vector yCE96 containing either the double mutant DT S508F S525F or a CRM9 COOH terminus fusion protein construct having reduced toxicity due to the COOH terminal added protein domain (Madshus et al. (1991)).

The mutated toxins are produced and purified analogously to the parent toxin except that low levels of reducing agent (equivalent to 2 mM betamercaptoethanol) are included in the purification to protect the unpaired introduced -SH group. Thioether chemical coupling is achieved to a single unpaired cysteine within the divalent antibody construct at either residue 414 in domain γCH3 or residue 575 in domain µCH4 when this domain is included. In this case domain γCH3 is mutated C414A to provide only a single coupling site. An advantage of including µCH4 is enhanced stability of the divalent antibody. A disadvantage is that the extra domain increases size and thereby reduces the secretion efficiency during antibody production. The advantage of terminating with the γCH3 domain is that, in another variant, a His6 purification tag can be added at either the µCH2 COOH or γCH3 COOH terminus to facilitate antibody purification. Another variant is to use the y hinge region to form the interchain disulfide and to couple through a γCH3 or µCH4. This variant has the advantage of being smaller in size and places the toxin moiety closer to the CD3 epitope binding domains, which could increase toxin membrane translocation efficiency. A His tag can be included at the carboxy terminus as a purification aid. SH-CRM9 is concentrated to 10 mg/ml in PBS pH 8.5 and reacted with a 15 fold molar excess of bismaleimidohexane (BMH) (Pierce, Rockford, IL). Excess BMH is removed by passing over a small G25F column (Pharmacia, Piscataway, NJ). The maleimide derived toxin at about 5 mg/ml is now added to scUCHT1 divalent antibody at 10 mg/ml at room temperature. After 1 hr the conjugate is separated from non-reactive starting products by size exclusion HPLC on a 5.08 cm by 25.4 cm (2 inch by 10 inch) MODcol column packed with Zorbax (DuPont) GF250 6 micron resin (for large scale production). Derivatives of ETA60EF61cys161 are also coupled to scUCHT1 divalent antibody by the same method.

Divalent anti-T cell fusion immunotoxins based on DT can be utilized in the invention, wherein the toxin domain (also referred to herein as "toxin moiety" or "tox") is either full length mutant S525F (CRM9) or truncated at 390 or 486 (collectively Tox) and the sequence of domains from the amino terminus from left to right can be selected from among the following and may include C terminal or amino terminal His purification tags: VL and VH are the variable light and heavy domains of the anti-CD3 antibody UCHT1 or other anti-CD3 antibody. H is the human IgG1 hinge.
Single chain divalent fusion protein: Tox, VL, L, VH, L, VL, L, VH;
Single chain univalent fusion protein homodimerized via µCH2 337 Cys: (Tox, VL, L, VH, µCH2)2;
Single chain univalent fusion protein homodimerized via H 226/229 Cys: (Tox, VL, L, VH, H, γCH3)2;
Single chain univalent fusion protein heterodimerized via µCH2 337 Cys: (Tox, VL, L, VH, µCH2
   VL, L, VH, µCH2);
Single chain univalent fusion protein heterodimerized via H 226/229 Cys: (Tox, VL, L, VH, H, γCH3
   VL, L, VH, H, γCH3);
sFv-SH fusion protein homodimerized via H 226/229 Cys chemically linked via a bis maleimide (R) to a SH derivatized CRM9 or a CRM9 containing an engineered C terminal cysteine (Tox): VL, L, VH, H, γCH3, His6, Cys-S-R-S-Tox.

Other types of protein toxin moieties can be utilized in anti-T cell immunotoxins for the induction of tolerance. All that is required is that a 1-2 log kill of T cells within the blood and lymph node compartments can be achieved without undue systemic toxicity. This in turn requires that the routing epitope routes in parallel with the toxin intoxication pathway and that binding site mutants are available or that toxins truncated in their binding domain are available that reduce toxin binding by 1000 fold compared to wild type toxins without compromising toxin translocation efficiency (see U.S. Patent No. 5,167,956 issued December 1, 1992). In addition when using targeting via antibodies, divalent antibodies are generally required under in vivo conditions to achieve sufficient cell killing due to the 3 to 15 fold lower affinity of monovalent antibodies. However, the method of linking the toxin to the divalent antibody either as a single chain fusion protein or through specific engineered coupling sites must not interfere with translocation efficiency. This could occur due to the larger size of many divalent antibodies compared to monovalent scFv antibodies unless care is taken so that the catalytic domain of the toxin can achieve unencumbered translocation. This is achieved for DT based immunotoxins using DT based binding site mutants where the fusion protein antibody moiety is contiguous with the COOH terminus of the toxin binding chain as described above. This allows the catalytic a chain to translocate as soon as the disulfide loop spanning the Arg/Ser proteolytic processing site residues 193/194 is reduced. Most targeted cells are capable of performing this processing event, and when chemically coupled CRM9 is used the processing is performed by trypsin prior to coupling.

If the toxin moiety is based on full length diphtheria toxin, it can include the following mutations:
S525F, K530C
S525F, K516C
S525F, D519C
S525F, S535C.

The antibody-toxin constructs utilized in the invention can be expected to be effective as immunotoxins because the relevant parameters are known. The following discussion of parameters is relevant to the use of the immunotoxin in tolerance induction. The relevant binding constants, number of receptors and translocation rates for humans have been determined and used. Binding values for anti-CD3-CRM9 for targeted and non-targeted cells in vitro and rates of translocation for the anti-CD3-CRM9 conjugate to targeted and non-targeted cells in vitro are described (Greenfield et al. (1987); Johnson et al. (1988); Johnson et al. (1989); Neville et al. (1989)). The rate limiting translocation rate to targeted cells in vitro is shown as follows: an anti-CD3-CRM9 conjugate at 10⁻¹¹ M is translocated to about 75% of the target cells present as measured by inhibition of protein synthesis in about 75% of cells with 20 hours. Inhibition of protein synthesis is complete in cells into which the conjugate translocates.

Parameters determined in in vivo studies in nude mice include the following: Tumor burden, as described as a constant mass equal to 0.1 % of body weight; the receptor number and variation of receptor number; "favorable therapeutic margin" as defined as an in vivo target cell 3 log kill at 0.5 MLD (minimum lethal dose) comparison of efficacy with an established treatment of 0.5 MLD immunotoxin equivalent to a radiation dose of 500-600 cGy.

The parameters determined in vitro allowed the prediction of success in the in vivo nude mouse study. Using the target cell number from the mouse study as being equivalent to the local T cell burden in a monkey or man successful T cell ablation and immunosuppression in monkeys could be predicted. Using the same parameters, a scientist skilled in this field can make a prediction of success in humans with confidence, because these parameters have been previously shown to have predictive success.

Most human sera contain anti-DT neutralizing antibodies from childhood immunization. To compensate for this the therapeutic dose of anti-CD3-CRM9 can be appropriately raised without affecting the therapeutic margin. Alternatively, a nontoxic DT mutants reactive with neutralizing antisera (e.g., CRM197) that can be administered in conjunction with the immunotoxin. See U.S. Patent No. 5,725,857.

Any one, two, or more of these adjunct therapies can be used together in the present method. Thus, the invention includes at least the following methods of preventing chronic rejection of a transplant in a recipient: (1) IT plus one costimulation blocker, for example, either an anti-CD154 or a blocker of the B7/CD28 interaction; (2) IT plus more than one costimulation blocker, for example, an anti-CD154 and a blocker of the B7/CD28 interaction; (3) IT plus one or more costimulation blockers and an immunosuppressant drug or combination of immunosuppressant drugs. The adjunct therapy can be administered before, at the same time or after the administration of immunotoxin. Different adjunct therapies can be administered to the recipient at different times or at the same time in relation to the transplant event or the administration of immunotoxin.

Because the immunosuppressant can be administered before the immunotoxin and/or other treatments, the present method can be used with a patient that has undergone an organ transplant and is on an immunosuppressant regimen. This presents a significant opportunity to reduce or eliminate traditional immunosuppressant therapy and its well documented negative side-effects. Also, as described below, treatment with immunosuppressants prior to transplantation could be particularly useful in cadaveric and xenogeneic transplants. In such a setting of pre-transplant treatment with immunosuppressant, the administration of immunotoxin can be delayed for up to seven or more days post-transplantation.

An example of a schedule of immunotoxin and immunosuppressant administration for patients receiving organ transplants is as follows:

| | |
|---|---|
| day -24 to -0 hours: | begin immunosuppressant treatment; |
| day 0 : | perform transplant; |
| day 0 : | immediately following transplant administer Ist immunotoxin dose; |
| day 1 : | 2nd immunotoxin dose; |
| day 2 : | 3rd and final immunotoxin dose. |

Immunosuppressant treatment may end at day 3 or extend to day 14. Immunosuppressant treatment is also effective if begun at the time of transplantation, and can continue for up to two weeks after transplantation. The costimulation blockers can be provided as described above in conjunction with the above regimen for IT and immunosuppressants.

The presently preferred doses of the immunotoxin are those sufficient to deplete peripheral blood T-cell levels to 80%, preferably 90% (or especially preferably 95% or higher) of preinjection levels. This should require mg/kg levels for humans similar to those for monkeys (e.g., 0.05 mg/kg to 0.3 mg/kg body weight), which toxicity studies indicate should be well tolerated by humans. Thus, the immunotoxin can be administered to safely reduce the recipients T cell population.

As used in the specification and in the claims, "a" can mean one or more, depending upon the context in which it is used.

The present invention is more particularly described in the following examples which are intended as illustrative only since numerous modifications and variations therein will be apparent to those skilled in the art.

### EXAMPLES

### EXAMPLE 1

### Combined Immunotoxin and Costimulation Blockade

*Animals.* Outbred 2.5-3.5 kg male or female rhesus *(Macaca mullata)* monkeys are obtained from the University of Wisconsin Primate Center (Madison, WI), LABS (Yemassee, SC) and other sources. Recipient and donor combinations as well as third-party animals are selected on the basis of MHC class I and class II disparity as determined by 1D-IEF gel electrophoresis and PCR-DGGE analysis and on the basis of one-way MLR. Animals are seronegative for simian immunodeficiency virus, simian T-cell lymphoma/leukemia virus, simian retrovirus and herpes B virus. Monkeys are also seronegative for anti-diphtheria toxin antibody.

*Surgery and animal care.* Heterotopic renal transplants are performed using the left kidney of the donor. Immediately after transplantation, the recipient undergoes native nephrectomy. Graft function can be monitored by measuring serum creatinine levels weekly. Rejection is determined by a rise in serum creatinine to > 8mg/dL, by the absence of technical problems (urine leak, obstruction) at autopsy, and by histological confirmation. A fine needle biopsy is taken under general anesthesia every month or every 6 months. Biopsies are analyzed histologically, by immunohistochemistry and by RT-PCR analysis using techniques well known in the art. (See, e.g., *Molecular Cloning,* eds. Sambrook, Fritsch, and Maniatis (1989; Armstrong et al. (1998)). Donor specific tolerance can be determined in long surviving animals by donor skin graft acceptance and third party skin graft rejection when animals are engrafted 5 - 6 months post renal transplant as previously described (Knechtle et al. 1997)). Nontolerant animals are defined as animals in which renal allograft and/or donor skin graft loss will be due to rejection.

*Immunosuppression.* FN18 antibody is chemically conjugated to CRM9 and purified as previously described to create anti-CD3-IT immunotoxin (Neville et al. (1992)). Starting on the day of transplantation, recipients receive a total dose of anti-CD3-IT immunotoxin of 0.2 mg/kg body weight given in 3 equal doses on three consecutive days. Animals to be given induction immunosuppression also receive methylprednisolone (250mg p.o.) on days 0, 1 and 2.

*Monitoring of T cell depletion and repopulation.* To prepare whole blood for flow cytometry, red blood cells are removed from whole blood by ACK lysis buffer (0.15 M NH₄Cl, 1.0 mM KHCO₃, 0.1 mM Na₂EDTA, pH 7.3) treatment prior to staining. Lymph node cells are prepared by emaceration and pressing through a fine stainless steel mesh. 1 x 105 cells are stained using standard protocols for a variety of cell surface markers including CD2, CD3, CD4, CD8, CD20, CD16, CD45RA or isotype control antibodies. Three-color flow cytometry are performed by staining cells with anti-CD3-biotin along with FITC and PE labeled antibodies and subsequent incubation with streptavidin PE-Cy5 (Waggoner et al. (1993)). Cells are then either subjected to flow cytometry immediately or fixed in 1% parafomaldehyde. Flow cytometry is performed using a single laser on a Bectin Dickenson FACSCAN. A minimum of 10,000 events are collected.

*MLR.* PBMC is isolated on Ficoll-Hypaque (Sigma) and washed three times in RPMI/10% FCS. PBMC is used either fresh or cyropreserved until the assay date. A monocyte-depleted responder population is obtained by passing cells over a Sephedex G-10 (Thomas et al. (1991)). 100µl stimulator cells and 100µl responder cells (each 05. x 106 cells /ml) are plate in 96 well round bottom culture plates. On days 3, 5 and 7, plates are harvested and counted after 16 hours labeling with 2.5 uCi/well 3H-thymidine.

*CTL LDA.* PBMC is isolated as described in the methods for MLR. Nonadherent responder PBMC are serially diluted, and 12 or 24 well replicates of each responder cell concentration is distributed into 96-well U-bottom plates, together with 5 x104 irradiated (3,000 rad) donor or third-party stimulator lymphocytes. The LDA for CTL is set up in RPMI plus 5% FCS + 5% autologous serum with 25 U/ml purified human rIL-2 (Collaborative Research, Bedford, MA) and culture period of 7-10 days. To determine CTL activity after the culture period, a standard 4 hour chromium release assay is performed. Briefly, PHA/IL2 -stimulated target lymphoblasts is labeled with 51 Cr and added to the wells at a density of 1 x 104 cells per well. Wells are scored as positive when 51 Cr-release was >3 SD above the mean spontaneous release.

*Statistics.* The CTLp frequency is calculated using the maximum likelihood method as outlined by Derry and Miller (1982). The paired comparisons of CTLp frequency, stimulation index and relative response as well as comparison of the pretransplant values of tolerant versus nontolerant animals for these measures is performed using Student's t-test. Fisher's exact test is used to determine any association between the probability of loss of CTLp or MLR response and the probability of becoming tolerant. Animals which are sacrificed for reasons other than graft loss due to rejection will be excluded from statistical analysis. Graft survival data are analyzed using the Weibull model as previously described (Van Houwelingen et al. (1995)).

Control groups consist of monkeys receiving IT alone, 5C8 alone, or CTLA4-Ig. In experimental groups, CTLA4-Ig or 5C8 is administered to monkeys in combination with a 3-day course of IT. The combination of immunotoxin and 5C8 and/or CTLA4-Ig is administered in two different manners: in one experimental group as a brief 1 week course (group 2) and in the second experiment as an extended protocol lasting 4 weeks (group 3). Ten monkeys are used in each group for statistical purposes, are shown below:
Group 1: IT alone day 0, 1, 2 (0.2 mg/kg)
Group 2: IT day 0, 1, 2 (0.2 mg/kg) + 5C8 day 0, 2, 4, 6, 8, 12, 16, 28 (20 mg/kg/day)
Group 3: 5C8 alone day 0, 2, 4, 6, 8, 12, 16, 28 (20 mg/kg/dose)
Group 4: CTLA4-Ig alone day 0, 2, 4, 6, 8, 12, 16, 28 (20 mg/kg/dose)
Group 5: IT day 0, 1, 2 (0.2 mg/kg) + CTLA4-Ig day 0, 2, 4, 6, 8, 12, 16, 28 (20 mg/kg/dose)
Group 6: 5C8 day 0, 1, 2 (0.2 mg/kg) + CTLA4-Ig day 0, 2, 4, 6, 8, 12, 16, 28 (20 mg/kg/dose)
Group 7: IT day 0, 1, 2 (0.2 mg/kg) + 5C8 day 0, 2, 4, 6, 8, 12, 16, 28 (20 mg/kg) + CTLA4-Ig day 0, 2, 4, 6, 8, 12, 16, 28 (20 mg/kg/dose)
Group 8: Untreated controls (n=4; 1 per year)

The technical failure rate is approximately 1/12 (n=5).

"Late" doses of costimulation blockade are given if anti-donor antibody titers rise at late time points. Renal transplant biopsies are performed monthly in half of all recipients and not at all in the other half, because the preliminary data suggest that biopsies may influence the outcome of the graft, perhaps by inducing nonspecific inflammation within the graft. All biopsies will be evaluated by H&E and silver stain to assess glomerular architecture. The phenotype of the graft infiltrating cells (GIC) will be evaluated by immunocytochemistry using antibodies for specific T cells (CD2, CD3, CD4, CD8) and B cells (CD20) as well as markers for macrophages and NK cells. In addition, analysis of cell death (programmed cell death/apoptosis versus active cytotoxicity) among GIC will be determined. Techniques will include immunocytochemistry to test for cytotoxic cell granule-related proteins (perforin, granzyme B, TIA-1) and in situ end labeling to assay apoptosis. (*See e.g.,* Gavrieli et al. (1992); Gorczyca et al. (1993); Wijsman et al. (1993); Li et al. (1995); Tornusciolo et al. (1995); Negoescu et al. (1996)).

### EXAMPLE 2

### In Vivo and In Vitro Tests of the Specificity of Combined Immunotoxin and Costimulation Blockade

*Tetanus and pneumococcal polysaccharide antigen immunization and measurement of subsequent humoral response.* Monkeys are immunized intramuscularly with alum adsorbed tetanus toxoid or pneumococcal polysaccharide antigen at either 1 month prior to transplant, at the time of transplant, 1 week post transplant and 2 months post transplant. Animals are bleed 1 week prior to immunization and for the first 4 weeks post immunization. Sera is measured for anti-tetanus or anti-pneumococcus IgG and IgM by ELISA (see below).

*ELISA for anti-mIgG, diphtheria toxin, tetanus toxoid and pneumococcal polysaccharide antigen.* 96 well flat bottom ELISA plates are coated overnight at 4°C with 25ml appropriate antigen (3mg/ml). After plates are blocked with PBS/2% BSA and washed, 25ml diluted serum is added to each well. After incubation for 1 hour at room temperature, plates are washed and appropriately diluted horseradish peroxidase-conjugated goat anti-human IgM or IgG is added to each plate. Again, after incubation for 1 hour at room temperature, substrate is added and, after adding stop solution, the plate is read by an automated ELISA plate reader. The titer is determined as the dilution with the absorbance reading less than 50% of maximal reading.

Immunotoxin, anti-CD154 and CTLA4-Ig each have a relatively long half-life and are non-specific (i.e., do not discriminate between allograft-specific and nonspecific immune cells). Thus, the effect of combined immunotoxin and costimulation blockade is assessed to determine if any resulting unresponsiveness or tolerance is universal or allograft specific and whether such unresponsiveness is long lived. Stated differently, the response of treated animals to infection can be assessed.

The immune response to a commonly studied protein antigen, namely tetanus antigen, can be studied in each of the groups of monkeys in Example 1. Both the primary and secondary immune response can be assessed by immunizing the monkeys in the following manner: 2 monkeys can receive their primary immunization 1 month pretransplant, 2 monkeys can be immunized on the day of transplant, 2 monkeys can be immunized 1 month posttransplant, and 2 monkeys immunized 3 months posttransplant. Both the primary immune response to tetanus immunization as well as the secondary response to a booster dose of tetanus antigen can be assessed in each monkey. The ability of monkeys to respond to allograft unrelated antigen such as infection while under treatment with costimulation blockers can thus be assessed.

A T cell independent antigen, namely pneumococcal polysaccharide antigen, can be used in a manner similar to that described for tetanus antigen to determine the monkey's response to a T cell-independent antigen. An immunization schedule analogous to that described above can be used with pneumococcal polysaccharide antigen. Such an antigen is a less potent stimulus of the immune system but is also an important measure of the monkey's ability to respond to infection.

As an *in vitro* correlate of the alloimmune response, all transplanted monkeys can be tested for their MLC and CTL responsiveness both to the donor lymphocytes and to two or more unrelated third-party monkeys. The purpose of this experiment is to determine the specificity of the immunosuppressive protocol and to assess how this may change over time. These tests can be performed pretransplant, and at 1, 3, 6, and 12 months posttransplant.

### EXAMPLE 3

### Donor-Specific Unresponsiveness following Combined Immunotoxin and Costimulation Blockade

*Detection of Anti-donor IgG.* Anti-donor antibodies (IgM or IgG) are detected by flow cytometry. Recipient serum is collected pretransplant, weekly for the first month and monthly thereafter. A minimum of 3 aliquots of 200µl each is stored at -85°C until assayed. Briefly, 50 µl of diluted recipient serum (starting at 1:10) is incubated with 50µl of donor or third-party PBL (2 x 107 cells/ml) on ice. After 30 minutes incubation, the cells is washed twice with PBS/1% FCS. Cells are then incubated in 100 µl PBS/1% FCS containing a FITC-conjugated goat anti-human IgG polyclonal antibody (Jackson ImmunoResearch Laboratories Inc.), diluted 1 :50 for 30, minutes. Cells are again washed twice and then analyzed using a FACSCAN (Becton-Dickenson) flow cytometer. Anti-T cell (Class I) and anti B cell (Class I and Class II) antibody specificity will be determined by dual staining of cells with anti-CD3 or anti-CD20 (Ohkawa et al. (1995)).

*Antibody-Complement-mediated Cytolysis (ACC) Assay.* ACC can be performed as previously described (Derry et al. (1982)). Target cells are 51Cr labeled donor and third party ConA blasts in RPMI/10% FCS. 10ml of target cells (1x 106/ml) are incubated with 20ml diluted serum in triplicate for 45 minutes in 96-well round bottom plates at room temperature. Rabbit compliment, diluted 1:8, is then added to each well (20 ml). Plates are incubated for 45 minutes at 37°C. After 100ml of cold media are added to each well, the plates are harvested and the supernatant is harvest on Skatron filters (Skatron, Sterling VA). Controls include spontaneous lysis (no compliment added) and maximum lysis (2% TritonX-100). Percent specific 51Cr release is then calculated.

One of the most rigorous *in vivo* tests of donor-specific unresponsiveness is skin grafting of a putatively tolerant recipient with donor and third-party skin. Monkeys from Example 1 can undergo donor and third-party skin grafting at 200 days posttransplant to determine whether monkeys develop, over time, donor-specific unresponsiveness and whether unresponsiveness to a renal allograft can be broken by exposure to a skin graft from the same donor.

Anti-donor IgG can be measured in all transplanted monkeys at a baseline time pretransplant and again every week for the first month and monthly thereafter. Under costimulation blockade, anti-donor antibody synthesis is downregulated. Two approaches to identify anti-donor antibodies can be used: (1) anti-donor mononuclear cell antibodies (principally against MHC antigens) will be measured and (2) kidney cells can be isolated from donor kidney biopsies and Western blot analysis used to identify antibodies to kidney cells.

### Reference EXAMPLE 4

### Rescue of Acute Allograft Rejection in Recipients Treated with Combined Immunotoxin and Costimulation Blockade

To determine whether failures (i.e., acute allograft rejection) in combined immunotoxin and costimulation blockade can be successfully rescued, the monkey renal allograft model can be used. Immunotoxin as a potent anti-T cell therapy can be used to rescue the allografts. Monkeys treated with any combination of immunotoxin and costimulation blockers who develop a rise in serum creatinine and have biopsy evidence of acute rejection can be treated with 3 doses of IT as rescue therapy. Follow-up creatinines, clinical exams, and biopsies can be used to assess whether acute rejection is reversed and whether the monkeys tolerates such therapy.

When monkeys treated with 5C8 and/or CTLA4-Ig develop an acute rejection, most do so within the first 200 days. Rejection is suspected if serum creatinine reaches 2.5 mg/dl and is confirmed by biopsy histology. Monkeys meeting these criteria can be treated with IT (0.2 mg/kg total dose) to reverse rejection. Criteria for successful rescue would be considered reversal of the rise in serum creatinine with subsequent return to baseline, histologic disappearance of tubulitis and other features of acute rejection, and absence of serious adverse effects of treatment.

### Reference EXAMPLE 5

### Human Studies Using Combined Immunotoxin and Costimulation Blockade

Because results in monkeys show that 5C8 by itself has significant efficacy in preventing acute allograft rejection, it can be used in humans following primary cadaveric renal transplants. The doses used can be those identified in current phase I clinical trials of 5C8 in patients with idiopathic thrombocytopenic purpura (ITP), an autoimmune disease or in a proposed phase I trial for patients with renal disease of an autoimmune nature (without transplantation). These two phase I trials of 5C8 make it unnecessary to plan a separate phase I trial for human kidney transplantation, especially since the kinetics of antibody clearance are not expected to be substantially different in patients with renal failure.

In phase II trials, either cyclosporine or prednisone can be deleted from the maintenance immunosuppressive therapy. Fifty patients can be enrolled in an open-label, nonrandomized, noncontrolled study to evaluate the safety and efficacy of 5C8 in preventing acute rejection and chronic rejection. 5C8 can be used as an induction agent in combination with maintenance immunosuppressive therapy consisting of either prednisone and mycophenolate or cyclosporine and mycophenolate. A single dose of 5C8 can be used and the drug can be administered on day 0, 2, 4, 6, 8, 12, 16, and 28.

Patient monitoring can include a hematologic survey (hemoglobin, RBC indices, WBC with differential, and platelet count) and a chemistry panel to include electrolytes, creatinine, BUN, INR, PTT, liver enzymes, LDH, amylase, and uric acid. These studies can be performed as a baseline before the first dose, and on days 1, 2, 3, and 4 following initial dosing, and then at 1, 2, 3, and 4 weeks posttransplant, and subsequently at 2 months, 3 months, 4 months, 6 months, 9 months, and 12 months. Additional monitoring can include a urinalysis, EKG, and chest x-ray as a baseline pre-dosing, and at 1 week, 1 month, 6 months, and 12 months. A physical exam can be performed at the beginning and at the conclusion of the study. Each patient can be followed for 12 months. Additional monitoring can include flow cytometry to evaluate CD3, CD4, CD8, CD20, and CD154 levels. These can be measured weekly during the first month and then at months 2, 3, 4, 6, 9, and 12. Expected toxic side effects can include weight loss. Outcomes to be monitored include acute and chronic rejection, infection, malignancy, graft function as reflected by serum creatinine and BUN, graft survival, and patient survival.

Because of the potential for 5C8 to prevent chronic rejection, biopsies can be performed on all subjects at two years and analyzed for histologic evidence of early changes of chronic rejection such as transplant glomerulopathy, arteriolar thickening, reduplication of the glomerular basement membrane, tubular atrophy, and interstitial fibrosis. In addition, cytokine analysis of graft infiltrating cells can be performed to determine whether cytokines predictive of chronic rejection are present. As a means of assessing whether chronic rejection is incipient and as a surrogate marker of histologic criteria for chronic rejection, kidney biopsies can be subjected to RNA extraction and PCR analysis of cytokine expression. Specifically, T-cell receptor turnover (CD3g), IL-2, 6, 8, and TNFa can be measured in biopsy specimens since these markers correlate with proteinuria, fibrosis, and tubular atrophy in results reported by (Kirk et al. (American Society of Transplant Physicians, 1997)). This methodology was developed by (Kirk et al. (Hum. Immunol. 1995), Kirk et al. (Transplantation 1997)). These clinical biopsy samples can be processed to extract RNA and make cDNA, and cytokines can be measured.

Although the present process has been described with reference to specific details of certain embodiments thereof, it is not intended that such details should be regarded as limitations upon the scope of the invention except as and to the extent that they are included in the accompanying claims.

### REFERENCES

Armstrong N, Buckley P, et al. Analysis of primate renal allografts following T-cell depletion with anti-CD3-CRM9. Transplantation 1998; 66:5-13.
Boussiotis VA, Gribben JG, Freeman GJ, Nadler LM. Blockade of the CD28 costimulatory pathway: a means to induce tolerance. Curr Opin Immunol 1994; 6:797.
Derry H, Miller RG. Measurement and calculation of CTL-P frequencies. In: Fathman CG, Fitch FW, eds. Isolation, Characterization and Utilization of T Lymphocyte Clones. New York: Academic Press, 1982; 501
Fechner JH Jr, Vargo DJ, Geissler EK, et al. Tolerance induced by immunotoxin in a rhesus kidney allograft model. Transplantation 1997; 63:1339.
Gavrieli Y, Sherman Y, Ben-Sasson SA. Identification of programmed cell death in situ via specific labeling of nuclear DNA fragmentation. J Cell Biol 1992; 119:493-501.
Greenfield et al., Mutations in Diphtheria Toxin Separate Binding from Entry and Amplify Immunotoxin Selectivity. Science 1987; 238:536-539.
Gorczya W, et al. Detection of DNA strand breaks in individual apoptotic cells by the in situ terminal deoxynucleotidyl transferase and nick translation assays. Cancer Res 1993; 53:1-7.
Grewal IS, Foellmer HG, Grewal KD, et al. Requirement for CD40 ligand in costimulation induction, T cell activation, and experimental allergic encephalomyelitis. Science 1996; 273:1864.
Johnson et al., Improved tumor-specific immunotoxins in the treatment of CNS and leptomeningeal neoplasia. J. Neurosurg 1989; 70:240-248.
Johnson et al., The Role of Diphtheria Toxin Receptor in Cytosol Translocation. J. Biol. Chem. 1988; 263 (3) 1295-1300.
Kaczorek M, Delpeyroux F, Chenciner N, Streek R (1983) Science; 221-885.
Kirk AD, Jacobson LM, Heisey DM, Fass NA, Sollinger HW, Pirsch JD. Posttransplant diastolic hypertension: associations with intragraft TGF-b, endothelin and renin transcription. Transplantation 1997; 64:1.
Kirk AD, Jacobson LM, Heisey DM, Fass NA, Pirsch JD, Sollinger HW. Biological monitoring of stable renal allografts: chronic rejection is subclinical acute rejection. American Society of Transplant Physicians 1997 Annual Meeting (abstract).
Kirk AD, Bollinger RR, Finn OJ. Rapid, comprehensive analysis of human cytokine mRNA and its application to the study of acute renal allograft rejection. Hum Immunol 1995; 43:113.
Knechtle SJ, Vargo D, Fechner JH Jr, et al. FN18-CRM9 immunotoxin promotes tolerance in primate renal allografts. Transplantation 1997; 63:1.
Lederman S., Yellin MJ, Krichevsky A, *et al.* (1992), Identification of a novel surface protein on activated CD4+T cells that induces contact-dependent B cell differentiation. *J. Exp. Med.* 175: 1091-1101.
Lederman.S., Yellin MJ, Inghirami G, *et al.* (1992), Molecular interactions mediating T-B lymphocyte collaboration in human lymphoid follicles: role of the T-cell-B-cell activating molecule (5c8 antigen) and CD40 in contact dependent help. *J*. *Immunonol.* 149:3817-3826.
Lenschow DJ, Zeng Y, Thistlethwaite JR, et al. Long-term survival of xenogeneic pancreatic islet grafts induced by CTLA4Ig. Science 1992; 257:789.
Li W, et al. Detection of apoptosis-associated DNA strand breaks by labeling 3'-OH termni with biotin- or digoxigenin-conjugated dUTP using terminal deoxynucleotidyl transferase. Biotechnic and Histochem 1995; 70:234-242.
Lin H, Bolling SF, Linsley PS, et al. Long-term acceptance of major histocompatibillity complex mismatched cardiac allografts induced by CTLA4Ig plus donor-specific transfusion. J Exp Med 1993; 178:1801-1806.
Linsley PS, Wallace PM, Johnson J, et al. Immunosuppression in vivo by a soluble form of the CTLA-4 T cell activation molecule. Science 1992; 257:792.
Madshus IH, Stenmark H, Snadvig K, Olsnes S (1991) J. Biol. Chem.; 266(26):17746-53.
Molecular Cloning. A Standard Laboratory Manual. 2nd Edition. Sambrook, J., Fritsch, E.F. and Maniatias, T. Eds. (1989) Cold Spring Harbour Laboratory Press.
Muhlrad D, Hunter R, Parker R (1992) Yeast; 8:79-82.
Negoescu A, et al. In situ apoptotic cell labeling by the TUNEL method: improvement and evaluation on cell populations. J. Histochem Cytochem 1996; 44:959-968.
Neville et al., Enhancement of Immunotoxin Efficacy by Acid-cleavable Cross-linking Agents Utilizing Diphtheria Toxin and toxin Mutants. J. Biol. Chem. 1989; 264 (25):14653-14661
Neville DM, Scharff J, Hu HZ, et al. A new reagent for the induction of T-cell depletion, anti-CD3-CRM9. J Immunother 1996; 19: 85.
Neville DM, Scharff J, Srinivasachar K. *In vivo* T-cell ablation by holo-immunotoxin directed at human CD3. Proc Natl Acad Sci USA 1992; 89: 2585.
Ohkawa S, Xu K, Wilson LA, Montelaro R, Martin LN, Murphey-Corb M. Analysis of envelope glycoprotein-specific antibodies from SIV-infected and gp 110-immunized monkeys in ACC and ADCC assays. AIDS Res Human Retroviruses 1995; 11:395.
Pearson et al. (1997), CTLA4-Ig plus bone marrow induces long-term allograft survival and donot specific unresponsiveness in the murine model: evidence for hematopoietic chimerism. Transplantation 61:997-1004.
Sayegh, MH and Turka, LA (1998), The role ofT-cell costimulatory activation pathways in transplant rejection. New England J. Med. 338:1813-1821.
Sayegh MH et al. (1997), Donor antigen is necessary for the prevention of chronic rejection in CTLA4-Ig-treated murine cardiac allograft recipients. Transplantation 64:1646-1650.
Schwartz RH. Models of T cell anergy: is there a common molecular mechanism? J Exp Med 1996; 184:1.
Shen WH, Choe S, Eisneberg D. Collier RJ (1994). Biol. Chem.; 469(46):29077-29084.
Thomas JM, Carver FM, Cunningham PRG, Olson LC, Thomas FT. Kidney allograft tolerance in primates without chronic immunosuppression: the role of veto cells. Transplantation 1991; 51: 198.
Tornusciolo D, et al. Simultaneous detection of TdT-mediated dUTP-biotin nick end labelling (TUNEL)- positive cells and multiple immunohistochemical markers in single tissue sections. Bio Techniques 1995; 19:800-805.
Van Houwelingen H, Thorogood J. Construction, validation and updating a prognostic model for kidney graft survival. Statistics in Medicine 1995; 14: 1999.
Waggoner AS, Ernst LA, Chen C-H, Rechtenwald DJ. A new fluorescent antibody label for three-color flow cytometry with a single laser. Ann NY Acad Sci 1993; 677:185.
Wijsman JH, et al. A new method to detect apoptosis in paraffin sections: in situ end-labeling of fragmented DNA. J Histochem Cytochem 1993; 41:7-12.

## Claims

1. Use of a combination of
a) an immunotoxin for reducing a recipient's T-cell population and
b) a costimulation blocker for reducing a transplant-specific antibody response
to prepare a medicament to prevent chronic rejection of a transplant in a recipient.

2. The use of claim 1, wherein the transplant is allogeneic.

3. The use of claim 1, wherein the recipient is a mammal.

4. The use of claim 3, wherein the mammal is a primate.

5. The use of claim 4, wherein the primate is a human.

6. The use of claim 1, wherein the immunotoxin transiently reduces the subject's T cells in the blood and lymph nodes by at least one log unit.

7. The use of claim 1, wherein the immunotoxin is a divalent anti-T cell immunotoxin directed at the CD3 epitope.

8. The use of claim 7, wherein the divalent anti-T cell immunotoxin comprises a toxin moiety and a targeting moiety directed to the T cell CD3ε epitope.

9. The use of claim 8, wherein the toxin moiety is a diphtheria toxin.

10. The use of claim 9, wherein the divalent anti-T cell immunotoxin is UCHT1-CRM9.

11. The use of claim 1, wherein the transplant is derived from a living donor.

12. The use of claim 1, wherein the transplant is derived from a cadaveric donor.

13. The use of claim 1, wherein the transplant is selected from the group consisting of kidney, liver, heart, pancreas, lung, and skin transplants.

14. The use of claim 1, wherein the costimulation blocker blocks the CD40:CD 154 pathway.

15. The use of claim 14, wherein the blocker of the CD40:CD154 pathway is an anti-CD154.

16. The use of claim 15, wherein the anti-CD154 is 5C8.

17. The use of claim 14, wherein the combination further comprises a second costimulation blocker, for further reducing the transplant-specific antibody response.

18. The use of claim 17, wherein the second costimulation blocker blocks the B7:CD28 pathway.

19. The use of claim 18, wherein the blocker of the B7:CD28 pathway is used to bind to B7 and to block binding of B7 with CD28.

20. The use of claim 19, wherein the blocker of the B7:CD28 pathway is CTLA 4-Ig.

21. The use of claim 1, wherein the costimulation blocker is used to block the B7:CD28 pathway.

22. The use of claim 21, wherein the blocker of the B7:CD28 pathway is CTLA 4-Ig.

23. The use of claim 1, wherein the combination further comprises an immunosuppressive agent.

24. The use of claim 23, wherein the immunosuppressive agent is selected from the group consisting of cyclosporine, mycophenolate moefitil, tacrolimus, azathioprine, and steroid and any combination thereof.

## Patentansprüche

1. Verwendung einer Kombination aus
a) einem Immunotoxin zur Reduktion der T-Zellpopulation eines Empfängers und
b) einem Co-Stimulierungsblocker zur Reduktion einer transplantatspezifischen Antikörperantwort
um ein Arzneimittel herzustellen, um die chronische Abstoßung eines Transplantats bei einem Empfänger zu verhindern.

2. Verwendung nach Anspruch 1, wobei das Transplantat allogen ist.

3. Verwendung nach Anspruch 1, wobei der Empfänger ein Säugetier ist.

4. Verwendung nach Anspruch 3, wobei das Säugetier ein Primat ist.

5. Verwendung nach Anspruch 4, wobei der Primat ein Mensch ist.

6. Verwendung nach Anspruch 1, wobei das Immunotoxin vorübergehend die T-Zellen bei dem Individuum in Blut und Lymphknoten um mindestens eine logarithmische Einheit reduziert.

7. Verwendung nach Anspruch 1, wobei das Immunotoxin ein divalentes Anti-T-Zellimmunotoxin ist, das gegen das CD3-Epitop gerichtet ist.

8. Verwendung nach Anspruch 7, wobei das divalente Anti-T-Zellimmunotoxin einen Toxinanteil und einen Anteil, der gezielt zum T-Zell-CD3ε-Epitop führt, aufweist.

9. Verwendung nach Anspruch 8, wobei der Toxinanteil ein Diphtherietoxin ist.

10. Verwendung nach Anspruch 9, wobei das divalente Anti-T-Zellimmunotoxin UCHT1-CRM9 ist.

11. Verwendung nach Anspruch 1, wobei das Transplantat von einem lebenden Spender stammt.

12. Verwendung nach Anspruch 1, wobei das Transplantat aus einem toten Spender stammt.

13. Verwendung nach Anspruch 1, wobei das Transplantat ausgewählt ist aus der Gruppe bestehend aus Nieren-, Leber-, Herz-, Pankreas-, Lungen- und Hauttransplantaten.

14. Verwendung nach Anspruch 1, wobei der Co-Stimulierungsblocker den CD40:CD154-Weg blockiert.

15. Verwendung nach Anspruch 14, wobei der Blocker für den CD40:CD154-Weg Anti-CD154 ist.

16. Verwendung nach Anspruch 15, wobei Anti-CD154 5C8 ist.

17. Verwendung nach Anspruch 14, wobei die Kombination weiterhin einen zweiten Co-Stimulierungsblocker aufweist, um die transplantatspezifische Antikörperantwort weiter zu senken.

18. Verwendung nach Anspruch 17, wobei der zweite Co-Stimulierungsblocker den B7:CD28-Weg blockiert.

19. Verwendung nach Anspruch 18, wobei der Blocker des B7:CD28-Wegs verwendet wird, um an B7 zu binden und die Bindung von B7 an CD28 zu blockieren.

20. Verwendung nach Anspruch 19, wobei der Blocker des B7:CD28-Wegs CTLA 4-lg ist.

21. Verwendung nach Anspruch 1, wobei der Co-Stimulierungsblocker verwendet wird, um den B7:CD28-Weg zu blockieren.

22. Verwendung nach Anspruch 21, wobei der Blocker des B7:CD28-Wegs CTLA 4-lg ist.

23. Verwendung nach Anspruch 1, wobei die Kombination weiterhin ein immunsupprimierendes Mittel enthält.

24. Verwendung nach Anspruch 23, wobei das immunsupprimierende Mittel ausgewählt ist aus der Gruppe bestehend aus Cyclosporin, Mycophenolatmoefitil, Tacrolimus, Azathioprin und Steroiden und jeder Kombination davon.

## Revendications

1. Utilisation d'une combinaison
a) d'une immunotoxine pour réduire la population de lymphocytes T d'un receveur et
b) d'un bloqueur de la costimulation pour réduire une réponse anticorps spécifique au greffon
pour préparer un médicament permettant de prévenir le rejet chronique d'un greffon chez un receveur.

2. Utilisation selon la revendication 1, dans laquelle le greffon est allogénique.

3. Utilisation selon la revendication 1, dans laquelle le receveur est un mammifère.

4. Utilisation selon la revendication 3, dans laquelle le mammifère est un primate.

5. Utilisation selon la revendication 4, dans laquelle le primate est un humain.

6. Utilisation selon la revendication 1, dans laquelle l'immunotoxine réduit de façon transitoire les lymphocytes T du sujet dans le sang et dans les ganglions lymphatiques d'au moins une unité log.

7. Utilisation selon la revendication 1, dans laquelle l'immunotoxine est une immunotoxine anti-lymphocytes T divalente dirigée contre l'épitope CD3.

8. Utilisation selon la revendication 7, dans laquelle l'immunotoxine anti-lymphocytes T divalente comprend une fraction ou partie toxique et une fraction ou partie ciblante dirigées contre l'épitope CD3ε des lymphocytes T.

9. Utilisation selon la revendication 8, dans laquelle la fraction ou partie toxique est une toxine diphtérique.

10. Utilisation selon la revendication 9, dans laquelle l'immunotoxine anti-lymphocytes T divalente est UCHT1-CRM9.

11. Utilisation selon la revendication 1, dans laquelle le greffon est issu d'un donneur vivant.

12. Utilisation selon la revendication 1, dans laquelle le greffon est issu d'un donneur décédé.

13. Utilisation selon la revendication 1, dans laquelle le greffon est choisi dans le groupe constitué par des greffons de rein, de foie, de coeur, de pancréas, de poumon et de peau.

14. Utilisation selon la revendication 1, dans laquelle le bloqueur de la costimulation bloque la voie CD40/CD 154.

15. Utilisation selon la revendication 14, dans laquelle le bloqueur de la voie CD40/CD154 est un anti-CD154.

16. Utilisation selon la revendication 15, dans laquelle l'anti-CD154 est 5C8.

17. Utilisation selon la revendication 14, dans laquelle la combinaison comprend en outre un second bloqueur de la costimulation pour réduire davantage la réponse anticorps spécifique au greffon.

18. Utilisation selon la revendication 17, dans laquelle le second bloqueur de la costimulation bloque la voie B7/CD28.

19. Utilisation selon la revendication 18, dans laquelle le bloqueur de la voie B7/CD28 est utilisé pour se lier à B7 et pour bloquer la liaison de B7 à CD28.

20. Utilisation selon la revendication 19, dans laquelle le bloqueur de la voie B7/CD28 est CTLA 4-Ig.

21. Utilisation selon la revendication 1, dans laquelle le bloqueur de la costimulation est utilisé pour bloquer la voie B7/CD28.

22. Utilisation selon la revendication 21, dans laquelle le bloqueur de la voie B7/CD28 est CTLA 4-Ig.

23. Utilisation selon la revendication 1, dans laquelle la combinaison comprend en outre un agent immunosuppresseur.

24. Utilisation selon la revendication 23, dans laquelle l'agent immunosuppresseur est choisi dans le groupe constitué par la cyclosporine, le mycophénolate de mofétil, le tacrolimus, l'azathioprine, et un stéroïde et toute combinaison de ceux-ci.
